# EUROPÄISCHE PATENTSCHRIFT

(11) **EP 3 222 296 B1**
(45) Veröffentlichungstag und Bekanntmachung des Hinweises auf die Patenterteilung: **09.01.2019**
(21) Anmeldenummer: 17162058.6
(22) Anmeldetag: 21.03.2017
(51) Int. Cl.: A61L 9/00, G05B 15/02

(54) **SYSTEM ZUR STEUERUNG EINER RAUMBEDUFTUNG**
SYSTEM FOR CONTROLLING THE FRAGRANCING OF A ROOM
SYSTÈME DE COMMANDE D'UNE DIFFUSION DE PARFUM DANS UN LOCAL

(30) Priorität: 22.03.2016 DE 102016105280
(43) Veröffentlichungstag der Anmeldung: 27.09.2017
(73) Patentinhaber: Deutsche Telekom AG, 53113 Bonn (DE)
(72) Erfinder: Huber, Michael, 67434 Neustadt a. d. W. (DE)
(74) Vertreter: Braun-Dullaeus Pannen Patent- und Rechtsanwälte

(56) Entgegenhaltungen:
- WO-A1-2008/142644
- WO-A1-2011/161643
- WO-A1-2015/130786

## Beschreibung

Die vorliegende Erfindung betrifft ein System zur Steuerung einer Raumbeduftung mit wenigstens einer Duftspendereinheit, die zur Abgabe verschiedener Duftrichtungen an einen Raum ausgebildet ist. Weiterhin betrifft die Erfindung eine Duftspendereinheit für ein solches System und ein Verfahren zur Steuerung einer Raumbeduftung.

### STAND DER TECHNIK

Aus der DE 195 13 293 A1 ist eine Duftspendereinheit bekannt, die mehrere Duftmodule umfasst. Mit jedem dieser Duftmodule kann eine bestimmte Duftrichtung in einen Raum abgegeben werden. Durch eine Mischung von Düften verschiedener Duftmodule können entsprechend mehr Duftrichtungen erzeugt werden. Aus der WO 2015/130786 A1, der WO 2008/142644 A1 und der WO 2011/161643 A1 sind Systeme bekannt, mit denen sich eine bestimmte Raumatmosphäre über die Einstellung verschiedener Parameter erzeugen lässt.

Raumbeduftungen werden heutzutage nicht nur zur Verbesserung der Luftqualität in einem Raum genutzt. Es ist hingegen auch bekannt, bestimmte Duftrichtungen einzusetzen, um Stimmungslagen bei Personen, beispielsweise im Rahmen verkaufsfördernder Maßnahmen, hervorzurufen. Zusätzlich zu bestimmten Stimmungen, die über Beleuchtungseinrichtungen oder Beschallungseinrichtungen erzeugt werden können, besteht mit Duftspendereinheiten zur Raumbeduftung die Möglichkeit, Stimmungsbilder bei Personen im Raum zu erzeugen oder eine Duftrichtung im Raum zu verbreiten, die von der steuernden Person ausgewählt wird. Diese Anpassung der Raumbeduftung kann zur Erhöhung des Wohlbefindens einer Person in einem Raum führen.

Darüber hinaus sind heutzutage sogenannte Smart-Home-Einrichtungen bekannt, die eine Art intelligente häusliche Umgebung bilden. Bei solchen Smart-Home-Einrichtungen erfolgt eine zumeist drahtlose Vernetzung von Haustechnik und Haushaltsgeräten, beispielsweise von Beleuchtungseinrichtungen, Beschallungseinrichtungen, jedoch auch Jalousien, Heizung, Küchengeräte oder beispielsweise Geräte der Unterhaltungselektronik. Die Smart-Home-Einrichtung kann mit einem meist mobilen Endgerät, beispielsweise einem Mobiltelefon, gesteuert werden, so dass eine Person über das handhaltbare Endgerät unmittelbaren Einfluss auf die elektronischen Komponenten innerhalb der Smart-Home-Einrichtung nehmen kann,

Beispielsweise offenbart die EP 2 890 056 A1 eine Smart-Home-Einrichtung, in der mehrere Komponenten über eine zentrale Instanz miteinander zu einem System verknüpft sind. Dem liegt der Gedanke zugrunde, eine Vielzahl unterschiedlicher Informationsquellen zur Situationserkennung zu nutzen und durch eine Vernetzung der von diesen Informationsquellen zur Verfügung gestellten Informationen eine Situationserkennung vorzunehmen sowie schließlich in Abhängigkeit des Ergebnisses der Situationserkennung Komponenten der intelligenten häuslichen Umgebung zu steuern oder einem Nutzer dieser Umgebung mit Ausgangsinformationen zu versorgen.

Aufgabe der Erfindung ist nunmehr die Weiterbildung eines Systems zur Steuerung einer Raumbeduftung, insbesondere um den Komfort einer häuslichen Umgebung für eine Person zu verbessern. Dabei soll eine Duftspendereinheit auf besonders bequeme Weise ansteuerbar sein.

Diese Aufgabe wird von einem System zur Steuerung einer Raumbeduftung gemäß Anspruch 1 und von einem Verfahren gemäß Anspruch 9 gelöst. Vorteilhafte Weiterbildungen der Erfindung sind in den abhängigen Ansprüchen angegeben.

Eine grundlegende Idee der Erfindung ist zunächst die Nutzung einer Smart-Home-Einrichtung zur Steuerung einer Duftspendereinheit vermittels insbesondere drahtloser Kommunikation. Dabei ist vermittels der Smart-Home-Einrichtung wenigstens eine Duftrichtung entsprechend einem vorgebbaren zu erzeugenden "Stimmungsbild" auswählbar und über die Duftspendereinheit an den Raum abgebbar.

Dabei sind die Stimmungsbilder ein zentraler Punkt der Erfindung. So sind Düfte im Gehirn mit Erinnerungen und/oder Empfindungen verbunden und setzen im Körper biochemische Mechanismen in Gang. Dabei werden die meisten Gerüche unbewusst wahrgenommen und steuern das Unterbewusstsein. Bei den Stimmungsbildern geht es nicht nur um bewußt wahrnehmbare Gerüche, sondern um unbewusst wahrgenommene Gerüche, die beim Menschen das Verhalten, die Stimmung, die Emotionen und/oder das Befinden beeinflussen. Selbst auf das Immunsystem und die Gesundheit wirken sich Gerüche aus. Erfindungsgemäß geht es also um mehr als um die Verbreitung eines angenehmen Geruchs. Dabei sind schon eine Vielzahl von Zusammenhängen zwischen einem unbewusst wahrgenommen Geruch und dem Effekt, den dieser beim Menschen auslöst, wissenschaftlich nachgewiesen. So verursacht beispielsweise Lavendel die Freisetzung von Serotonin, das beruhigt, während Minze als Stimmungsaufheller und erfrischend wirkt. Zitrusaromen wirken konzentrationsfördernd und anregend. Pheromone verursachen hingegen die Freisetzung von dem Glückshormon Dopamin. Oxytocin erzeugt Vertrauen und soziale Nähe.

Der wesentliche Kerngedanke der Erfindung liegt somit nicht nur in der Einbindung der Duftspendereinheit in eine Smart-Home-Einrichtung, sondern auch in der Erzeugung eines gewünschten Stimmungsbildes über die von der Duftspendereinheit automatisch entsprechend eines vom Nutzer ausgewählten Stimmungsbildes abgegebene Duftnote.

Durch die erfindungsgemäße Einbindung kann die Duftspendereinheit auf vorteilhafte Weise angesteuert werden, sodass die Ausgabe einer bestimmten Duftrichtung entsprechend dem gewünschten Stimmungsbild nicht an der Duftspendereinheit selbst manuell eingestellt werden muss. Zur Erzeugung eines bestimmten Stimmungsbildes kommuniziert die Smart-Home-Einrichtung mit der Duftspendereinheit in der Weise, dass das gewünschte Stimmungsbild vorgewählt werden kann. Die Duftspendereinheit wird entsprechend angesteuert und "komponiert" automatisch den entsprechenden Duft zu dem gewünschten Stimmungsbild. Somit sind beim Benutzer keine besonderen Kenntnisse über den Zusammenhang eines Stimmungsbildes und seiner Erzeugung durch eine Duftnote erforderlich. Die Erzeugung der Duftrichtungen wird automatisch über die Smart-Home-Einrichtung anhand hinterlegter Rezepturen vorgenommen.

Somit liegt ein wesentlicher Gedanke der Erfindung darin, das Wissen der Geruchsspezialisten zu automatisieren und für den Nutzer nur noch eine Bedienoberfläche über eine App mit auswählbaren Stimmungsbildern für unterschiedliche Räumlichkeiten auswählbar machen. Die Hausautomatisation über Smart-Home und der zugehörigen App erledigt dann den Rest. Somit wird je nach eingestelltem "Stimmungsbild" automatisch der zugehörige Duft aus dem Multi-Duftspende freigesetzt.

Nach einer vorteilhaften Weiterführung des erfindungsgemäßen Systems ist ein handhaltbares Endgerät, insbesondere ein Smartphone mit entsprechender Applikation, vorgesehen, das zur drahtlosen Kommunikation mit der Smart-Home-Einrichtung eingerichtet ist und mit dem die Duftspendereinheit über die Smart-Home-Einrichtung steuerbar ist. Dabei kann eine Person sowohl innerhalb des Raumes, beispielsweise innerhalb eines Hauses, oder auch außerhalb des Hauses eine Steuerung der Smart-Home-Einrichtung vornehmen, wenn die Person das handhaltbare Endgerät bei sich trägt. Mit dem Endgerät, insbesondere dem Smartphone, kann die Person die Smart-Home-Einrichtung so ansteuern, dass sie über eine Applikation ein Stimmungsbild auswählt, wobei die Applikation die Duftspendereinheit zur Beduftung des Raumes mit einer entsprechenden Duftnote aktiviert. Die Auswahl kann über verschiedene fest vorgegebene oder über frei beschreibbare Stimmungsbilder erfolgen. Die ensprechende Duftnote wird automatisch erzeugt.

Die Applikation (App) auf dem Smartphone ist so ausgebildet, dass das gewünschte Stimmungsbild einfach von der Person durch eine Auswahl aus verschiedenen Stimmungsbildern vorgegeben werden kann. Die Beduftung des Raumes erfolgt dann automatisch und in einer Intensität, die für die Person lediglich unterbewusst wahrnehmbar ist und zu dem gewünschten Stimmungsbild führt. So können Stimmungsbilder gezielt beeinflusst werden, beispielsweise um ein Wohlgefallen der Person im Raum zu erzeugen, ohne dass die Person dies aktiv mit einer Duftrichtung im Raum in Verbindung bringt.

Gemäß einer vorteilhaften Weiterbildung des Systems ist wenigstens eine Beleuchtungseinrichtung zur Beleuchtung des Raumes und/oder eine Beschallungseinrichtung zur Beschallung des Raumes vorgesehen, welche Beleuchtungseinrichtung oder Beschallungseinrichtung ebenfalls mittels der Smart-Home-Einrichtung steuerbar ist, wobei die Steuerung der Beleuchtungseinrichtung und/oder der Beschallungseinrichtung an die Ansteuerung der Duftspendereinheit mit Bezug auf ein Stimmungsbild der Person anpassbar ist. Damit kann die Duftspendereinheit eingebunden werden in ein System mehrere elektronischer Geräte im Verbund der Smart-Home-Einrichtung, und die Duftspendereinheit kann auf ähnliche Weise angesteuert werden wie beispielsweise eine Beleuchtungseinrichtung oder eine Beschallungseinrichtung. Dabei sieht das System insbesondere eine Anpassung einer abzugebenden Duftrichtung vor, die harmoniert mit einer erzeugten Beleuchtung des Raumes oder einer ausgewählten Beschallung des Raumes.

Mit weiterem Vorteil weist die Duftspendereinheit ein Kommunikationsmodul zur drahtlosen Kommunikation mit der Smart-Home- Einrichtung auf. Das Kommunikationsmodul bildet beispielsweise ein Funkmodul, das zur drahtlosen Kommunikation mit der Smart-Home-Einrichtung kommunizieren kann.

Die Erfindung richtet sich weiterhin auf eine Duftspendereinheit für ein System zur Steuerung einer Raumbeduftung wie vorstehend beschrieben. Die Duftspendereinheit weist mehrere Duftmodule auf, die zur Abgabe unterschiedlicher Düfte ausgebildet sind. Die Duftmodule können austauschbar in einem Grundkörper der Duftspendereinheit aufgenommen sein, und die Duftspendereinheit kann beispielsweise über das Kommunikationsmodul an die Smart-Home-Einrichtung die Information übermitteln, ob die Duftmodule beispielsweise aufgrund des Verbrauchs von Duftstoffen ausgetauscht werden müssen. Jedes Duftmodul kann sich auch mit Inhalt und Füllstand identifizieren, so dass die steuernde Person über die App erkennen kann, welche Stimmungsbilder anhand der Bestückung im jeweiligen Raum machbar sind.

Mit weiterem Vorteil weist die Duftspendereinheit ein Steckerelement auf, mit dem die Duftspendereinheit in einer Haussteckdose einsetzbar ist und womit eine Stromversorgung der Duftspendereinheit eingerichtet werden kann. Zusätzlich oder alternativ kann die Duftspendereinheit ein Batteriefach zur Aufnahme von Batterien oder Akkus aufweisen.

Die Erfindung richtet sich weiterhin auf ein Verfahren zur Steuerung einer Raumbeduftung, mit wenigstens einer Duftspendereinheit, mit der verschiedene Duftrichtungen an einen Raum abgegeben werden können. Erfindungsgemäß wird die Duftspendereinheit mit einer Smart-Home-Einrichtung angesteuert, sodass mittels der Smart-Home-Einrichtung wenigstens eine Duftrichtung aus verschiedenen möglichen Duftrichtungen ausgewählt und über die Duftspendereinheit an den Raum abgegeben wird. Erfindungsgemäß wird an die Smart-Home-Einrichtung ein gewünschtes Stimmungsbild einer Person übermittelt, wobei die Person das Stimmungsbild aktiv übermitteln kann und an die Smart-Home-Einrichtung übermittelt. Schließlich kann mit der Smart-Home-Einrichtung die Duftspendereinheit so angesteuert werden, dass ein an das Stimmungsbild angepasster Raumduft in den Raum abgegeben wird.

### BEVORZUGTES AUSFÜHRUNGSBEISPIEL DER ERFINDUNG

Weitere, die Erfindung verbessernde Maßnahmen werden nachstehend gemeinsam mit der Beschreibung bevorzugter Ausführungsbeispiele der Erfindung anhand der Figuren näher dargestellt. Es zeigt:
- Figur 1: eine schematische Ansicht des erfindungsgemäßen Systems zur Steuerung einer Raumbeduftung mit einer Smart-Home-Einrichtung und mit einer beispielhaft gezeigten Duftspendereinheit und
- Figur 2: eine schematische Ansicht einer Duftspendereinheit mit den Merkmalen der vorliegenden Erfindung.

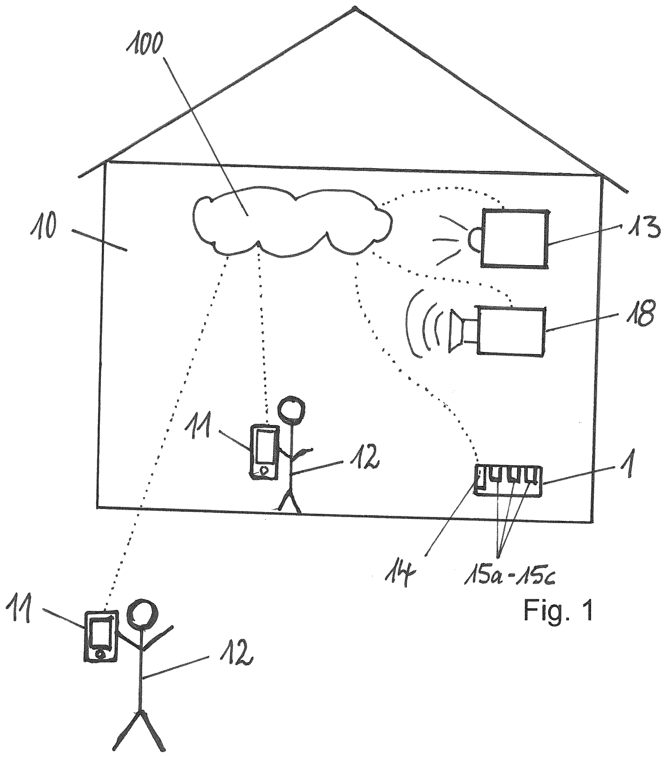

Figur 1 zeigt ein Haus zur Bildung eines Raumes 10, in dem eine Duftspendereinheit 1 aufgenommen ist. Die Duftspendereinheit 1 dient zur Erzeugung und Abgabe einer Duftrichtung in den Raum 10 entsprechend eines ausgewählten Stimmungsbildes. Weiterhin ist eine Smart-Home-Einrichtung 100 gezeigt, die als drahtloses Haus-Kommunikationssystem aufgebaut ist und mit der mehrere Geräte, beispielsweise also auch eine Beleuchtungseinrichtung 13 und eine Beschallungseinrichtung 18 verbunden werden können. Insbesondere ist gemäß der Erfindung die Duftspendereinheit 1 mit der Smart-Home-Einrichtung 100 verbunden.

Eine erste Person 12 ist beispielhaft außerhalb des Raumes 10 gezeigt und eine zweite Person 12 ist innerhalb des Raumes 10 gezeigt. Im Besitz der Personen 12 befinden sich Endgeräte 11, die beispielsweise durch Mobiltelefone, insbesondere Smartphone gebildet werden.

Mittels der Endgeräte 11 können die Personen 12 mit der Smart-Home-Einrichtung 100 kommunizieren, um die technischen Einrichtungen des Hauses beispielsweise zu aktivieren, anzusteuern oder zu programmieren. Erfindungsgemäß wird die Duftspendereinheit 1 drahtlos mit der Smart-Home-Einrichtung 100 angesteuert beziehungsweise die Duftspendereinheit 1 ist Bestandteil der Smart-Home-Einrichtung 100. Zur Kommunikation mit der Smart-Home-Einrichtung 100 weist die Duftspendereinheit 1 ein Kommunikationsmodul 14 auf, über das eine Kommunikation stattfinden kann zwischen der Smart-Home-Einrichtung 100 und der Duftspendereinheit 1. Die Duftspendereinheit 1 weist mehrere Duftmodule 15a, 15b und 15c auf, und jedes der Duftmodule 15a, 15b oder 15c dient zur Abgabe einer bestimmten Duftrichtung in oder an den Raum 10. Dabei können beispielsweise drei oder mehr Duftmodule 15a, 15b, 15c vorhanden sein, beispielsweise kann die Duftspendereinheit 1 auch fünf oder zehn Duftmodule aufweisen. Insbesondere können die Duftmodule 15a, 15b, 15c auch in unterschiedlichen Intensitäten gleichzeitig aktiviert werden, sodass Duftmischungen entstehen, und sodass mehr als nur beispielsweise drei Duftrichtungen von der Duftspendereinheit 1 erzeugt werden können.

Die Smart-Home-Einrichtung 100 ist so ausgelegt, dass diese beispielsweise über eine App, die auf dem Endgerät 11 betrieben werden kann. Dabei kann die Person 12 eine zu erzeugende Stimmungslage festlegen. Angepasst an die ausgewählte Stimmungslage wird die Duftspendereinheit 1 mit der Smart-Home-Einrichtung 100 so angesteuert, dass eine entsprechende Duftrichtung in den Raum 10 durch die Duftspendereinheit 1 abgegeben wird, die die gewünschte Stimmungslage erzeugt. Dabei kann sich die Person 12 bereits im Raum 10 aufhalten oder die Person 12 befindet sich außerhalb des Raumes 10 und es ist zu erwarten, dass die Person 12 in absehbarer Zeit den Raum 10 betritt, beispielsweise wenn die Person 12 nach Hause kommt.

Wenn die steuernde Person beispielsweise gestresst ist, kann sie das Stimmungsbild "Entspannung" auswählen, wozu vorteilhafterweise der Duft von Lavendel eingesetzt wird.

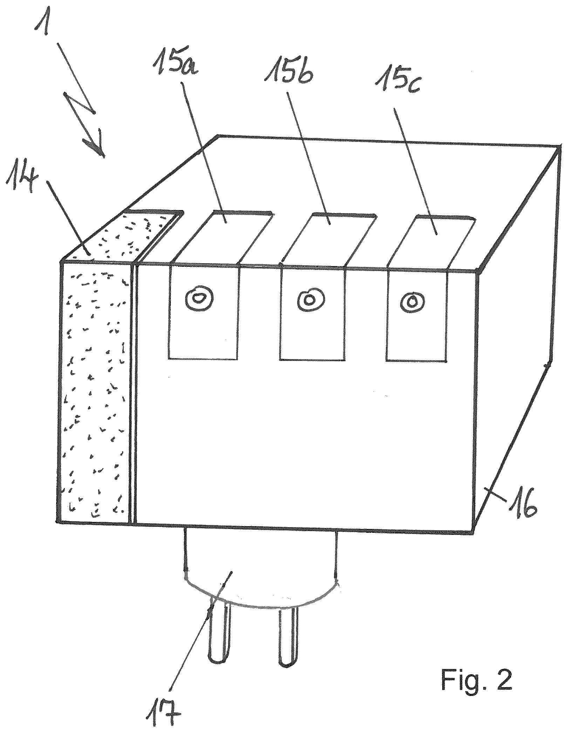

Figur 2 zeigt eine vergrößerte Ansicht einer Duftspendereinheit 1 mit den Merkmalen der vorliegenden Erfindung. Die Duftspendereinheit 1 weist einen Grundkörper 16 auf, an dem sich ein Steckerelement 17 befindet, und mit dem die Duftspendereinheit 1 beispielsweise in einer Haussteckdose einsteckbar ist, um eine Stromversorgung der Duftspendereinheit 1 sicherzustellen. Am Grundkörper 16 der Duftspendereinheit 1 befinden sich beispielhaft gezeigt drei Duftmodule 15a, 15b, 15c, und jedes der Duftmodule 15a, 15b, 15c dient zur Abgabe einer bestimmten Duftrichtung. Jedes Duftmodul identifiziert sich mit Inhalt und Füllstand, damit kann die steuernde Person über die App erkennen welche Stimmungsbilder anhand der Bestückung im jeweiligen Raum machbar sind. Weiterhin weist die Duftspendereinheit 1 ein Kommunikationsmodul 14 auf, mit dem die Duftspendereinheit 1 mit der Smart-Home-Einrichtung 100 kommunizieren kann.

Auf nicht näher gezeigte Weise besteht auch die Möglichkeit, dass die Duftspendereinheit 1 unmittelbar mit dem Endgerät 11 kommunizieren kann, ohne über die Smart-Home-Einrichtung 100 zu kommunizieren. Somit kann die Person 12 beispielsweise mit dem Endgerät 11 die Duftspendereinheit 1 aktivieren und bestimmte Düfte freisetzen.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei grundsätzlich anders gearteten Ausführungen Gebrauch macht. Sämtliche aus den Ansprüchen, der Beschreibung oder den Zeichnungen hervorgehenden Merkmale und/oder Vorteile, einschließlich konstruktiver Einzelheiten oder räumlicher Anordnungen, können sowohl für sich als auch in den verschiedensten Kombinationen erfindungswesentlich sein. Insbesondere können mehrere Duftspendereinheiten 1 im Raum 10 vorgesehen sein, die jeweils mit der Smart-Home-Einrichtung 100 in Verbindung stehen. Auch besteht die Möglichkeit, dass die Duftspendereinheit 1 mehr als nur drei Duftmodule 15a, 15b, 15c aufweist.

### Bezugszeichenliste

- 100: Smart-Home-Einrichtung

- 1: Duftspendereinheit

- 10: Raum
- 11: Endgerät
- 12: Person
- 13: Beleuchtungseinrichtung
- 14: Kommunikationsmodul
- 15a: Duftmodul
- 15b: Duftmodul
- 15c: Duftmodul
- 16: Grundkörper
- 17: Steckerelement
- 18: Beschallungseinrichtung

## Patentansprüche

1. System zur Steuerung einer Raumbeduftung, mit wenigstens einer Duftspendereinheit (1), die zur Abgabe verschiedener Duftrichtungen an einen Raum (10) ausgebildet ist, wobei eine Smart-Home-Einrichtung (100) vorgesehen und die Duftspendereinheit (1) zur drahtlosen Kommunikation mit der Smart-Home-Einrichtung (100) ausgebildet ist, wobei ein handhaltbares Endgerät (11) vorgesehen ist, das zur drahtlosen Kommunikation mit der Smart-Home-Einrichtung (100) eingerichtet ist und mit dem die Duftspendereinheit (1) über die Smart-Home-Einrichtung (100) steuerbar ist, **dadurch gekennzeichnet, dass** mittels des Endgerätes (11) ein Stimmungsbild ausgewählt wird und an die Smart-Home-Einrichtung (100) vorgegeben wird, wobei mittels der Smart-Home-Einrichtung (100) eine dem Stimmungsbild entsprechende Duftrichtung aus verschiedenen Duftrichtungen anhand hinterlegter Rezepturen erzeugt wird und über die Duftspendereinheit (1) als Beduftung an den Raum (10) abgegeben wird, wobei die Beduftung automatisch und in einer Intensität erfolgt, die für eine Person lediglich unterbewußt wahrnehmbar ist und zu dem gewünschten Stimmungsbild führt.

2. System nach Anspruch 1, **dadurch gekennzeichnet, dass** das handhaltbare Endgerät (11) ein Smartphone ist.

3. System nach einem der Ansprüche 1 oder 2, **dadurch gekennzeichnet, dass** das Endgerät (11) eine Applikation zur Vorgabe des Stimmungsbildes aufweist, wobei mittels der Applikation die Duftspendereinheit (1) über die Smart-Home-Einrichtung (100) steuerbar ist.

4. System nach einem der vorgenannten Ansprüche, **dadurch gekennzeichnet, dass** wenigstens eine Beleuchtungseinrichtung (13) zur Beleuchtung des Raumes (10) und/oder eine Beschallungseinrichtung (18) zur Beschallung des Raumes (10) vorgesehen ist, welche Beleuchtungseinrichtung (13) oder Beschallungseinrichtung (18) mittels der Smart-Home-Einrichtung (100) steuerbar ist, wobei die Steuerung der Beleuchtungseinrichtung (13) und/oder der Beschallungseinrichtung (18) an die Ansteuerung der Duftspendereinheit (1) mit Bezug auf ein Stimmungsbild der Person (12) anpassbar ist.

5. System nach einem der vorgenannten Ansprüche, **dadurch gekennzeichnet, dass** die Duftspendereinheit (1) ein Kommunikationsmodul (14) zur drahtlosen Kommunikation mit der Smart-Home-Einrichtung (100) aufweist.

6. System nach einem der vorgenannten Ansprüche, **dadurch gekennzeichnet, dass** die Duftspendereinheit (1) mehrere Duftmodule (15a, 15b, 15c) aufweist, die zur Abgabe unterschiedlicher Düfte ausgebildet sind.

7. System nach Anspruch 6, **dadurch gekennzeichnet, dass** die verschiedenen Duftmodule (15a, 15b, 15c) austauschbar in einem Grundkörper (16) der Duftspendereinheit (1) aufgenommen sind.

8. System nach einem der Ansprüche 6 oder 7, **dadurch gekennzeichnet, dass** ein Steckerelement (17) vorgesehen ist, mit dem die Duftspendereinheit (1) in eine Haussteckdose einsetzbar ist und womit eine Stromversorgung der Duftspendereinheit (1) erzeugbar ist.

9. Verfahren zur Steuerung einer Raumbeduftung mittels eines Systems nach einem der vorherigen Ansprüche aufweisend wenigstens eine Duftspendereinheit (1), mit der verschiedene Duftrichtungen an einen Raum (10) abgegeben werden, wobei die Duftspendereinheit (1) mit einer Smart-Home-Einrichtung (100) angesteuert wird, wobei ein handhaltbares Endgerät (11) vorgesehen ist, das zur drahtlosen Kommunikation mit der Smart-Home-Einrichtung (100) eingerichtet ist und mit dem die Duftspendereinheit (1) über die Smart-Home-Einrichtung (100) gesteuert wird, **dadurch gekennzeichnet, dass** mittels des Endgerätes (11) ein Stimmungsbild ausgewählt und an die Smart-Home-Einrichtung (100) vorgegeben wird, wobei mittels der Smart-Home-Einrichtung (100) eine dem Stimmungsbild entsprechende Duftrichtung aus verschiedenen Duftrichtungen anhand hinterlegter Rezepturen erzeugt und über die Duftspendereinheit (1) als Beduftung an den Raum (10) abgegeben wird, wobei die Beduftung automatisch und in einer Intensität erfolgt, die für eine Person lediglich unterbewußt wahrnehmbar ist und zu dem gewünschten Stimmungsbild führt.

## Claims

1. System for controlling a room fragrancing device with at least one fragrances diffuser unit (1) which is designed to diffuse different fragrances into a room (10), wherein a smart home device (100) is provided and the fragrance diffuser unit (1) is designed for wireless communication with the smart home device (100), wherein a handheld terminal device (11) is envisaged which is configured for wireless communication with the smart home device (100) and with which the fragrance diffuser unit (1) can be controlled via the smart home device (100), **characterised in that** by means of the terminal device (11) an ambience can be selected and specified to the smart home device (100), wherein by means of the smart home device (100) a fragrance corresponding to the ambience is produced from different fragrances by way of stored recipes and diffused into the room (10) as a fragrance via the fragrance diffuser unit (1), wherein fragrancing takes place automatically and at an intensity which is only subconsciously perceived by a person and results in the desired ambience.

2. System according to claim 1 **characterised in that** the portable terminal device (11) is a smart phone.

3. System according to any one of claims 1 or 2 **characterised in that** the terminal device (11) has an application for specifying the ambience, wherein by way of the application the fragrance diffuser unit (1) can be controlled via the smart home device (100).

4. System according to any one of the preceding claims **characterised in that** at least one lighting system (13) for lighting the room (10) and/or sound system (18) for filling the room (10) with sound is/are provided, said lighting system (13) or sound system (18) being controllable via the smart home device (100), wherein the control of the lighting system (13) and/or the sound system (18) is matchable to the control of the fragrance diffuser unit (1) in relation to the ambience selected by the person (12).

5. System according to any one of the preceding claims **characterised in that** the fragrance diffuser unit (1) comprises a communication module (14) for wireless communication with the smart home device (100).

6. System according to any one of the preceding claims **characterised in that** the fragrance diffuser unit (1) comprises several fragrance modules (15a, 15b, 15c) designed for diffusing different fragrances.

7. System according to claim 6 **characterised in that** the different fragrance modules (15a, 15b, 15c) are exchangeably accommodated in a main body (16) of the fragrance diffuser unit (1).

8. System according to any one of claims 6 or 7 **characterised in that** a plug element (17) is provided with which the fragrance diffuser unit (1) can be inserted into a household socket and with which a power supply to fragrance diffuser unit (1) can be generated.

9. Method of controlling the fragrancing of a room by means of a system according to any one of the preceding claims comprising at least one fragrance diffuser unit (1) with which different fragrances are diffused into a room (10), wherein the fragrance diffuser unit (1) is controlled with a smart home device (100), wherein a handheld terminal device (11) is provided which is configured for wireless communication with the smart home device (100) and with which the fragrance diffuser unit (1) is controlled by way of the smart home device (100), **characterised in that** by means of the terminal device (11) an ambience is selected and specified to the smart home device (100), wherein by way of the smart home device (100) a fragrance corresponding to the ambience is produced by means of stored recipes and via the fragrance diffuser unit (1) is diffused as a fragrance into the room (10), wherein the fragrancing takes place automatically and at an intensity which the person only perceives subconsciously and results in the desired ambience.

## Revendications

1. Système, destiné à commander une diffusion de parfum d'ambiance, comprenant au moins un module diffuseur de parfum (1), qui est conçu pour la distribution de différents parfums dans une pièce (10), un système de maison intelligente (100) étant prévu et le module diffuseur de parfum (1) étant conçu pour la communication sans fil avec le système de maison intelligente (100), un terminal portable (11) étant prévu, qui est agencé pour la communication sans fil avec le système de maison intelligente (100) et à l'aide duquel le module diffuseur de parfum (1) est susceptible d'être commandé via le système de maison intelligente (100), **caractérisé en ce qu'**au moyen du terminal (11), un type d'ambiance est choisi et prescrit au système de maison intelligente (100), au moyen du système de maison intelligente (100), un parfum correspondant au type d'ambiance étant créé à partir de différents parfums, à l'aide de formules sauvegardées et étant distribué via le module diffuseur de parfum (1) pour parfumer la pièce (10), la distribution de parfum s'effectuant automatiquement et selon une intensité uniquement perceptible par le subconscient d'une personne et qui mène vers le type d'ambiance souhaité.

2. Système selon la revendication 1, **caractérisé en ce que** le terminal portable (11) est un smartphone.

3. Système selon l'une quelconque des revendications 1 ou 2, **caractérisé en ce que** le terminal (11) comporte une application pour la prescription du type d'ambiance, au moyen de l'application, le module diffuseur de parfum (1) étant susceptible d'être commandé via le système de maison intelligente (100).

4. Système selon l'une quelconque des revendications précédemment citées, **caractérisé en ce qu'**il est prévu au moins un système d'éclairage (13), destiné à éclairer la pièce (10) et/ou un système de sonorisation (18), destiné à sonoriser la pièce (10), lequel système d'éclairage (13) ou système de sonorisation (18) étant susceptible d'être commandé au moyen du système de maison intelligente (100), la commande du système d'éclairage (13) et/ou du système de sonorisation (18) étant adaptable au module diffuseur de parfum (1), en rapport à l'état d'esprit de la personne (12).

5. Système selon l'une quelconque des revendications précédemment citées, **caractérisé en ce que** le module diffuseur de parfum (1) comporte un module de communication (14) pour la communication sans fil avec le système de maison intelligente (100).

6. Système selon l'une quelconque des revendications précédemment citées, **caractérisé en ce que** le module diffuseur de parfum (1) comporte plusieurs modules de parfums (15a, 15b, 15c) qui sont conçus pour distribuer différents parfums.

7. Système selon la revendication 6, **caractérisé en ce que** les différents modules de parfum (15a, 15b, 15c) sont réceptionnés de manière interchangeable dans un corps de base (16) du module diffuseur de parfum (1).

8. Système selon l'une quelconque des revendications 6 ou 7, **caractérisé en ce qu'**il est prévu un élément connecteur (17), par lequel le module diffuseur de parfum (1) est insérable dans une prise domestique et à l'aide duquel une alimentation en courant électrique du module diffuseur de parfum (1) peut être créée.

9. Procédé, destiné à commander un diffuseur de parfum d'ambiance au moyen d'un système selon l'une quelconque des revendications précédentes, comportant au moins un module diffuseur de parfum (1), à l'aide duquel différents parfums sont distribués dans une pièce (10), le module diffuseur de parfum (1) étant actionné à l'aide d'un système de maison intelligente, un terminal portable (11) étant prévu, qui est agencé pour la communication sans fil avec le système de maison intelligente (100) et à l'aide duquel le module diffuseur de parfum (1) est commandé via le système de maison intelligente (100), **caractérisé en ce qu'**au moyen du terminal (11), un type d'ambiance est sélectionné et prescrit au système de maison intelligente (100), au moyen du système de maison intelligente (100), un parfum correspondant au type d'ambiance composé de différents parfums étant créé à l'aide de formules sauvegardée et distribué via le module diffuseur de parfum (1) pour parfumer la pièce (10) la distribution de parfum s'effectuant automatiquement et selon une intensité uniquement perceptible par le subconscient d'une personne et menant vers le type d'ambiance souhaité.
